# EUROPEAN PATENT SPECIFICATION

(11) **EP 2 365 985 B1**
(45) Date of publication and mention of the grant of the patent: **15.05.2013**
(21) Application number: 09799602.9
(22) Date of filing: 15.12.2009
(51) Int. Cl.: C07K 14/745, C07K 1/18, C07K 1/22

(54) **PURIFICATION OF FACTOR V**
AUFREINIGUNG VON FAKTOR V
PURIFICATION DU FACTEUR V

(30) Priority: 16.12.2008 US 201942 P; 16.12.2008 EP 08171792
(43) Date of publication of application: 21.09.2011
(73) Proprietor: Crucell Holland B.V., 2333 CN Leiden (NL)
(72) Inventor: VERSCHUUR, Maartje, NL-2333 CN Leiden (NL); HEEMSKERK, Evert, NL-2333 CN Leiden (NL); MARKUS, Paul, Henri, NL-2333 CN Leiden (NL); VAN MIERLO, Gerardus, Jacobus, NL-2333 CN Leiden (NL)
(86) International application number: PCT/EP2009/067159
(87) International publication number: WO 2010/069946

(56) References cited:
- US-A- 5 219 995
- BOS M H A ET AL: "DOES ACTIVATED PROTEIN C-RESISTANT FACTOR V CONTRIBUTE TO THROMBIN GENERATION IN HEMOPHILIC PLASMA?" JOURNAL OF THROMBOSIS AND HAEMOSTASIS, BLACKWELL PUBLISHING, OXFORD, GB, vol. 3, no. 3, 1 March 2005 (2005-03-01), pages 522-530, XP008078605 ISSN: 1538-7933 cited in the application
- KANE W H ET AL: "Purification and characterization of human coagulation factor V." THE JOURNAL OF BIOLOGICAL CHEMISTRY 25 JAN 1981, vol. 256, no. 2, 25 January 1981 (1981-01-25), pages 1002-1007, XP002513003 ISSN: 0021-9258
- PFLEGERL K ET AL: "Mutational analysis of a blood coagulation factor VIII-binding peptide" JOURNAL OF PEPTIDE RESEARCH, BLACKWELL PUBLISHING LTD., OXFORD, GB, vol. 59, no. 4, 1 April 2002 (2002-04-01), pages 174-182, XP002434462 ISSN: 1397-002X
- KATZMANN J A ET AL: "ISOLATION OF FUNCTIONAL HUMAN COAGULATION FACTOR V BY USING A HYBRIDOMA ANTIBODY" PROCEEDINGS OF THE NATIONAL ACADEMY OF SCIENCES OF THE USA, NEW YORK, NY, US, vol. 78, no. 1, 1 January 1981 (1981-01-01), pages 162-166, XP002048309
- SILVEIRA JAY R ET AL: "Purification and partial characterization of platelet factor V suggest it is physically distinct from plasma factor V" BLOOD, vol. 96, no. 11 Part 1, 16 November 2000 (2000-11-16), page 635a, XP009111433 & 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- AMATSCHEK KARIN ET AL: "Affinity chromatography of human blood coagulation factor VIII on monoliths with peptides from a combinatorial library" HRC JOURNAL OF HIGH RESOLUTION CHROMATOGRAPHY, vol. 23, no. 1, January 2000 (2000-01), pages 47-58, XP002513131 ISSN: 0935-6304
- BRANOVIC K ET AL: "Application of semi-industrial monolithic columns for downstream processing of clotting factor IX" JOURNAL OF CHROMATOGRAPHY B: BIOMEDICAL SCIENCES & APPLICATIONS, ELSEVIER, AMSTERDAM, NL, vol. 790, no. 1-2, 25 June 2003 (2003-06-25), pages 175-182, XP004426798 ISSN: 1570-0232
- BERTINA R M ET AL: "MUTATION IN BLOOD COAGULATION VACTOR V ASSOCIATED WITH RESISTANCE TO ACTIVATED PROTEIN C" NATURE, NATURE PUBLISHING GROUP, LONDON, UK, vol. 369, no. 6475, 5 May 1994 (1994-05-05), pages 64-67, XP000563812 ISSN: 0028-0836
- BARUT M ET AL: "Methacrylate-based short monolithic columns: Enabling tools for rapid and efficient analyses of biomolecules and nanoparticles" JOURNAL OF SEPARATION SCIENCE 200806 DE, vol. 31, no. 11, June 2008 (2008-06), pages 1867-1880, XP002513132 ISSN: 1615-9306 1615-9314

## Description

The invention relates to the field of proteins, in particular to the purification of proteins. More in particular, the invention relates to the purification of blood coagulation Factor V (FV).

### Background of the invention

Factor V is a major player in the coagulation cascade leading to the formation of the fibrin clot through the action of thrombin. Activated Factor V (FVa) acts as a cofactor for the serine protease Factor Xa (FXa), enhancing conversion of prothrombin to thrombin by five orders of magnitude. The half-life of FVa is down regulated by activated protein C (APC) that cleaves FVa at several postions in the heavy chain of the protein. Cleavage at two postions, R306 and R506, was shown to be the major contributor to the inactivation of FVa by APC. The APC resistant double mutant of FV, FV-Leiden/Cambridge that harbors the R306T and R506Q mutations, increases the lifetime of this protein and therefore is a potential therapeutic candidate in treatment of blood disorders involving low thrombin generation (WO2008/059009).

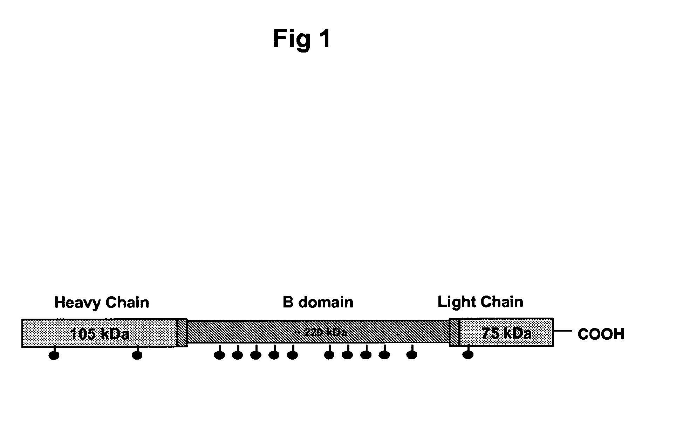

Full length FV is a 330 KDa polypeptide with a domain structure similar to Factor VIII (FVIII). After proteolytic activation by thrombin to form FVa, the protein is composed of a heavy chain and a light chain non-covalently associated with a calcium binding site at the interface between the two chains. FV contains multiple posttranslational modifications such as glycosylations, sulfations and phosphorylations (qualitatively represented by black dots in FIG. 1) that are important for the cofactor function. The glycosyl groups give about 13% of the total molecular weight of FV with a high degree of sialylation.

Research grade protocols for the purification of Factor V have been described by several authors, both for the purification of Factor V from human or bovine plasma (Neshheim et al.) and for the purification ofrecombinant Factor V produced on cell cultures (vd Neut et al., Bos et al.). In general, these protocols have not been designed with the purpose of pharmaceutical production, and cannot readily be used for this goal. For instance, the protocol described by Bos et al contains the following steps:
1. Concentration of cell culture harvest using an "artificial kidney" (Hemoflow F5 hollow fiber from Fresenius, Bad Homburg, Germany).
2. Purification of Factor V by immuno-affinity using a monoclonal antibody specific for Factor V directed against the B-domain (a-FV); performed in batch mode.
3. Buffer exchange by dialysis.
4. Further concentration by anion exchange chromatography.
5. Buffer exchange into the 50% glycerol-based storage buffer by dialysis.

The main drawbacks of this protocol are the long process time and the fact that the protocol includes process steps that are not suitable for scale-up (i.e. initial concentration step, buffer exchange step by dialysis). Other protocols disclosed hitherto have similar drawbacks.

There remains a need in the art for further possibilities to purify Factor V. More specifically, there remains a need for industrial processes for Factor V purification. It is the object of the present invention to provide alternative methods for purification of Factor V.

### Summary of the invention

The invention provides a method for purifying coagulation Factor V from a biological fluid comprising, in the given order, the steps of: a) binding factor V to an anion exchanger; b) washing the anion exchanger with a first solution to remove contaminants; c) eluting Factor V; d) specifically binding factor V to a matrix containing anti-Factor V antibodies; e) washing said matrix containing anti-Factor V antibodies with a second solution to remove contaminants; f) eluting Factor V.

In one embodiment, the anion exchanger used in step a) is a filter. In another embodiment the anion exchanger used in step a) is a chromatographic monolith containing groups with high anion exchanger functionality, such as quaternary amine groups.

In further embodiments, the elution in step c) is performed by treating the anion exchanger with a buffer containing between 0.3 and 1 M NaCl.

In further embodiments, the matrix used in step d) is an immuno-affinity capture filter membrane. In preferred embodiments, the matrix used in step d) is a cross-linked polystyrene-divinylbenzene matrix to which epoxide functional groups are bound.

The disclosure further provides the use of a chromatographic monolith containing quaternary amine groups for the purification of coagulation Factor V from a biological fluid.

The disclosure further provides the use of a chromatographic monolith containing quaternary amine groups for the separation of active Factor V from inactive Factor V, wherein the active form of Factor V is at least two times as active as the inactive form of Factor V in a clot activity assay.

In certain embodiments, the coagulation Factor V has been recombinantly expressed. In further embodiments, the coagulation factor V is an APC-resistant Factor V mutant.

### Brief description of the Figures

FIG. 1: Domain structure of Factor V molecule.
FIG. 2: Schematic overview of the clot activity assay.
FIG. 3: Process flow diagram of Factor V purification process based on research-grade protocol described by Bos et al.
FIG. 4: Process flow diagram of Factor V purification process according to the invention (Anion exchange followed by Immuno-affinity).
FIG. 5: Process flow diagram of Factor V purification process according to the invention with the use of a chromatographic monolith.
FIG. 6: SDS-Page of eluate fractions from the anion exchanger (monolith) and the immuno affinity step.

### Detailed description of the invention

The present invention provides a scalable process for Factor V purification with a reduced process time compared to processes developed hitherto. The invention resides in the use of an anion exchange step followed by an immuno-affinity step. This particular order allows for high flow capacity and a reduced process time compared to previously designed processes such as for instance, the process described by Bos et al. Moreover, the current process enables the separation of active Factor V from inactive Factor V.

Compared to Bos et al. the novel process of the present invention is initiated with an anion exchange (AEX) step, wherein factor V is concentrated. Also, during this step, DNA as well as contaminants are removed from the biological fluid. Subsequently, the biological fluid is, either directly or following a dilution, further processed on an immuno-affinity matrix containing anti-Factor V antibodies.

Surprisingly this resulted in the possibility to omit the initial concentration step as well as the dialysis step from the process described in Bos et al. and therewith a significant time reduction could be achieved. The omission of the concentration and dialysis steps, which are steps that are not scalable, allow for the current process of this invention to be scaled-up and therefore to be applicable in an industrial process, in contrast to the process in Bos et al.

It was also disclosed herein that the anion exchange step is performed with a specific chromatographic monolith, which has a high resolution. A two step gradient elution allows for the separation of active Factor V from inactive Factor V.

Several properties of Factor V, as well as the possibility to use it for hemostatic treatment, are for instance described in WO2008/059009, incorporated herein in its entirety by reference. The term "Factor V" as used herein encompasses within its meaning human Factor V, such as wild type human plasma Factor V (SEQ ID NO: 1 in WO2008/059009), as well as natural allelic variations thereof, but also Factor V variants containing one or more amino acid alterations by deletion, substitution or addition, and chemically modified Factor V. Several examples of such molecules are given in WO2008/059009. For instance, one or more amino acids of Factor V, preferably not more than 30, more preferably not more than 20, still more preferably not more than 10, most preferably zero, one, two or three amino acids, may be replaced with other amino acids, or eliminated, or added. Typically, molecules that are at least 70%, preferably at least 80%, more preferably at least 90%, still more preferably at least 95% identical in amino acid sequence to SEQ ID NO: 1 of W02008/059009 is encompassed within the term Factor V as used herein. In certain embodiments, Factor V has the amino acid sequence of wild type human plasma Factor V. The term "Factor V" also includes APC-resistant FV mutants (Bertina et al, 1994, Svensson et al 1994, Williamson et al, 1998 and Chan, 1998). These mutants are inactivated more slowly by APC and hence prolong the activity of Factor Xa. Thus, via their effect on FXa these FV mutants enhance thrombin formation. APC-resistant FV cannot act as a cofactor for APC and thus lacks the anti-coagulant effect of its wild type counterpart. In preferred embodiments, FactorV according to the present invention therefore is APC-resistant Factor V (WO2008/059009, WO2008/059043).

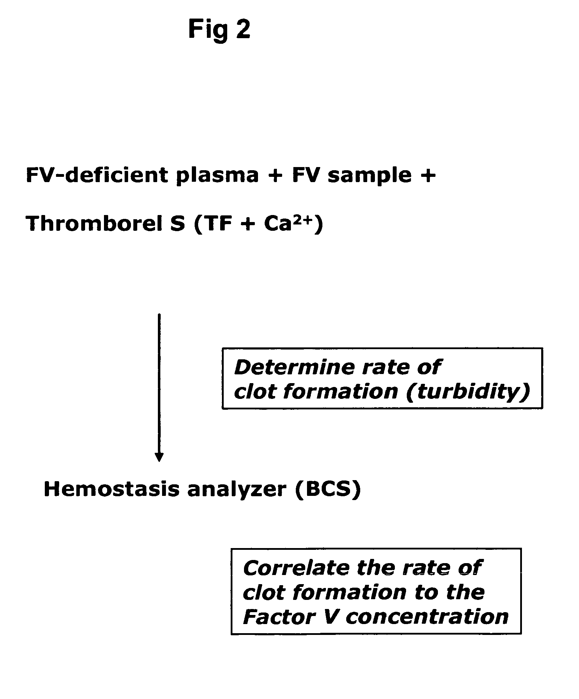

Factor V, according to the present invention, exists either in an active or inactive form. Said active form induces clot formation while the inactive form is not capable of inducing clot formation. The ability to induce clot formation is tested in a clot activity assay, which is based on a prothrombin time (PT) assay performed using FV-deficient human plasma. The clot activity assay determines the concentration of active Factor V contained in a preparation by correlating the clotting time (time for clot formation induced by factor V) to the Factor V concentration. FIG. 2 shows a schematic view of the clot activity assay and example 3 provides a suitable clot activity assay. The assay was performed according to methods known to the person skilled in the art.

Factor V is purified according to the invention from a biological fluid. A biological fluid may be any fluid derived from or containing cells, cell components or cell products. Biological fluids include, but are not limited to cell cultures, cell culture supernatants, cell lysates, cleared cell lysates, cell extracts, tissue extracts, blood, plasma, serum, milk, urine, plant extracts and fractions thereof, all of which may also be homogenizates and filtrates, and fractions thereof, for instance collected by chromatography of unfractionated biological fluids.

Factor V may be purified from a wide variety of biological fluids, including cell culture supernatants, which naturally produce Factor V, but preferably of cells which have been genetically modified to produce recombinant Factor V, such as mammalian cells e.g. Chinese hamster ovary (CHO) cells, HEK293 cells, BHK cells, PER.C6 cells (as deposited at the ECACC under no. 96022940; for recombinant expression of proteins in PER.C6 cells see e.g. US patent 6,855,544), yeast, fungi, insect cells, and the like, or prokaryotic cells, or transgenic animals or plants. In certain embodiments, recombinant expression is achieved in PER.C6 cells that further over-express a sialyltransferase, e.g. human α-2,3-sialyltransferase (see e.g. WO2008/059043, WO2008/059009). Methods for recombinant expression of desired proteins are known in the art, and recombinant production of for instance APC-resistant FV has been described in e.g. EP 0756638 and WO2008/059009.

In one embodiment, the biological material is derived from human blood plasma. In a preferred embodiment, the biological material is derived from a culture of cells in which Factor V is recombinantly expressed, for instance a cell culture supernatant or cell-conditioned culture medium thereof.

Cell culture supernatants or cell conditioned culture medium in the capture step of Factor V according to the invention may be derived from cells grown in the presence of serum such as fetal calf serum, or grown in serum free medium. Cell conditioned culture medium denotes a nutrient medium in which cells have been cultured and which contains cell products. When working with biological fluids containing cells, cell debris and the like it is preferred to first filter and/or (ultra)centrifuge the fluid to remove particulate contaminants. Recombinant Factor V is secreted by the cells into the cell culture medium (cell-conditioned culture medium), or present in cell lysates, and can be separated according to the invention from other cell components, such as cell waste products, cell debris and proteins or other collected material.

Purification of Factor V is the process of increasing the concentration of Factor V (enriching) in a sample in relation to other components of said sample, resulting in an increase of the purity of Factor V. The increase in purity of Factor V may be followed by use of methods known in the art, such as for instance by use of SDS-PAGE, HPLC or ELISA. Purification is done to remove undesired contaminants, and therewith increasing the purity of Factor V. The term purified as used herein in relation to a protein does not refer only to absolute purity (such as a homogeneous preparation); instead, it refers to a protein that is relatively purer than in the natural environment. A step of purifying a protein thus relates to obtaining a protein preparation in which the protein is purer than in the biological material prior to the purification step, i.e. the preparation contains less contaminants, contaminants in this context including other proteins than Factor V.

The present invention provides a process for purification of Factor V, which comprises an anion exchange chromatography step followed by an immuno-affinity step.

Ion exchange chromatography (see e.g. GE Healthcare, "Ion exchange chromatography & chromatofocusing", Principles & Methods" Cat. no. 11-0004-21) relies on charge interactions between the protein of interest and the ion exchange matrix, which is generally composed of a solid support, such as agarose, dextran, cross-linked cellulose, and the like, covalently bound to a charged group. Charged groups are classified according to type (cationic and anionic) and strength (strong or weak); the charge characteristics of strong ion exchange media do not change with pH, whereas with weak ion exchange media, sample loading and capacity can change owing to loss of charge at varying pH, preventing protein binding. Examples of commonly used charged groups include diethylaminoethyl (DEAE; weak anionic exchanger), carboxymethyl (weak cationic exchanger), quaternary ammonium (strong anionic exchanger), and methyl sulfonate (strong cationic exchanger). Other charged groups are available as well. Ion exchange resins selectively bind proteins of opposite charge; that is, a negatively charged resin will bind proteins with a positive charge and vice versa. The technique in general takes place in five steps: equilibration of the column to pH and ionic conditions ideal for target protein binding; reversible adsorption of the sample to the column through counterion displacement; introduction of elution conditions that change the buffer's pH or ionic strength in order to displace bound proteins; elution of substances from the column in order of binding strength (weakly bound proteins are eluted first); and re-equilibration of the column for subsequent purifications. The skilled person can design ion exchange chromatography protocols such that the target protein is selectively bound to the column (allowing contaminants to pass through) or so that contaminants adsorb and the target protein is excluded. In addition to resins that can be used in batch or to prepare columns, ion exchange can also be performed using high throughput ion exchange membranes/filters (i.e. a charged membrane or filter that contains ion exchange groups). Such ion exchange filters are known in the art and are commercially available, e.g. from Pall (e.g. Mustang^{™} series) and from Sartorius (e.g. Sartobind series). Such filters can have advantages compared to ion exchange columns, for instance in example 2 it is shown that filters allow for higher flow rates, resulting in a reduced process time.

Therefore in one preferred embodiment of the present invention, the anion exchanger is a filter containing a cellulose matrix with quaternary ammonium functional groups bound to it.

Currently, a new generation of columns for ion exchange chromatography has been developed based on the CIM® (Convective interaction media) technology. The technology relies on large inner channel diameter and convective mass transfer. The CIM® monolithic supports are based on a highly cross-linked porous monolithic polymer, such as polyglycidyl methacrylate-co-ethylene dimethacrylate or polystyrene-divinylbenzene polymers with well-defined, bimodal channel-size distribution, which allow for high flow rates.

In one preferred embodiment of the present invention the anion exchanger is a chromatographic monolith, which in certain embodiments is based on a polyglycidyl methacrylate-co-ethylene dimethacrylate matrix with quaternary amine (QA) functional groups. Herewith, the process time was reduced similarly as with the use of filters. Additionally, said chromatographic monolith could unexpectedly separate active Factor V from inactive Factor V, as shown in example 3. The anion exchanger used in the present method is therefore preferably a chromatographic monolith containing quaternary amine groups. Such chromatographic monoliths are known in the art and commercially available, e.g. from BIA separations (e.g. CIM ® QA monolithic column).

During the anion exchange chromatography step, the negatively charged Factor V proteins are bound to positively charged functional groups on the surface of the anion exchanger. This anion exchange chromatography step may be used for DNA removal as well. Since host cell DNA is negatively charged it will be bound to functional groups on the surface of the chromatographic support.

Subsequent to the anion exchange chromatography step, the anion exchanger is washed with a buffer with high buffer capacity at pH value between about 5 and 9, preferably at pH value between about 6 and 8 (e.g. Tris, HEPES). In certain embodiments of the present invention, the buffer contains between 100 mM and 200 mM NaCl; between 0.5 mM and 5 mM CaCl₂ and between 1% and 20% glycerol. In a preferred embodiment said buffer contains of 150 mM NaCl; 1 mM CaCl₂ and 10% glycerol. Additionally, said buffer contains between 5 and 50 mM benzamidine, which is a protease inhibitor. Preferably, said buffer contains 10 mM benzamidine. The anion exchanger is washed in order to remove impurities.

Elution of factor V from the anion exchanger is achieved e.g. by increasing the ionic strength of the buffer, in particular by increasing the sodium chloride concentration. The optimal sodium chloride concentration is dependent on the type of anion exchanger. The person skilled in the art is able to optimize the sodium chloride concentration in order to obtain maximal Factor V elution. In certain embodiments of the present invention, the NaCl concentration in the elution buffer lies between about 0.5 and 1 M NaCl, for instance when the anion exchanger is a filter. Preferably the NaCl concentration in the elution buffer is about 0.6 M when the anion exchanger is a filter. In other embodiments of the present invention the NaCl concentration in the elution buffer lies between 0.30 and 1 M NaCl for instance when the anion exchanger is a chromatographic monolith. Unexpectedly, it appeared that when applying a two step buffer gradient on the chromatographic monolith, active Factor V could be separated from inactive Factor V. In a certain embodiment of the present invention, the NaCl concentration in the buffer used to elute active Factor V is between about 0.35 and 0.5 M. Preferably said concentration is between about 0.35 and 0.4 M. Even more preferably, said concentration is about 0.36 M. In yet another embodiment of the present invention, the NaCl concentration in the buffer used to elute inactive Factor V is between about 0.5 and 1 M. Preferably said concentration is between about 0.5 and 0.6 M. Even more preferably, said concentration is about 0.56 M.

It was disclosed herein that a chromatographic monolith can be used for the separation of active from inactive Factor V. Preferably a chromatographic monolith containing quaternary amine groups is used for the separation of active from inactive Factor V.

the active form of Factor V as purified using a monolith is at least two times as active as the inactive form of Factor V in a clot activity assay.

In a later stage, DNA is eluted from the anion exchanger. This process requires a higher ionic strength compared to Factor V proteins. Therefore, material eluted from an anion exchanger at ionic strength suitable for Factor V elution will not contain host cell DNA.

According to the present invention, the anion exchange step is followed by an immuno-affinity step. The previously eluted fraction of Factor V is brought into contact with an immuno-affinity chromatography matrix.

Immunoaffinity chromatography is a specialized form of affinity chromatography (see e.g. Affinity Chromatography, Principles & Methods. GE Healthcare, Cat.no. 18-1022-29) and, as such, utilizes an antibody or antibody fragment as a ligand immobilized onto a solid support matrix in a manner that retains its binding capacity. Immunoaffinity chromatography relies on the highly specific interaction of an antigen with its antibody. The highly selective loops on the antibody surface capture the antigen with high affinity, while having little interaction with impurities and other components that may also be present in the biological fluid.

In the present invention, the immuno-affinity chromatography matrix contains anti-Factor V antibodies, which bind specifically to Factor V. In certain embodiments said anti-Factor V antibodies are covalently bound to an epoxide functional group, which is coupled to the support matrix.

In certain embodiments of the present invention, said support matrix is an immuno-affinity capture filter membrane. These membranes can consist of a cellulose matrix with epoxide functional groups as for instance the Sartobind Epoxy 75 from Sartorius.

In one embodiment of the present invention, said support matrix is a cross-linked polystyrene-divinylbenzene matrix. In a preferred embodiment said support matrix consists of cross-linked polystyrene-divinylbenzene flow through particles. These particles are coated with a cross-linked polyhydroxylated polymer, which are then activated with epoxide functional groups. Cross-linked polystyrene-divinylbenzene matrix with epoxide groups are commercially available e.g. from Applied biosystems (e.g. POROS® series).

During the immuno-affinity chromatography step, the Factor V proteins are specifically bound to anti-Factor V antibodies. Subsequent to the immuno-affinity chromatography step, the immuno-affinity matrix is washed with a buffer preferably at pH value between 6 and 8 (e.g. Tris, HEPES). In certain embodiments of the present invention, said buffer is a Tris buffer which contains between 100 mM and 300 mM NaCl; between 0.5 mM and 5 mM CaCl₂ and between 5% and 20% ethylene glycol. In a preferred embodiment said buffer consists of 200 mM NaCl; 1 mM CaCl₂ and 10% ethylene glycol. The immuno-affinity matrix is washed in order to remove impurities.

Elution of factor V from the immuno-affinity matrix was achieved e.g. by increasing the ionic strength of the buffer, in particular by increasing the sodium chloride concentration. The optimal sodium chloride concentration is dependent on the type of the immuno-affinity matrix. The person skilled in the art knows how to optimize the sodium chloride and ethylene glycol concentration in order to obtain maximal Factor V elution. In certain embodiments of the present invention, the NaCl concentration in the elution buffer is between about 1,5 M and 3 M. The ethylene glycol concentration lies between 40% and 60%. Preferably the NaCl concentration in the elution buffer is 2 M and the ethylene glycol concentration is 50%.

In preferred embodiments, the Factor V purification process of the invention gives a yield of at least 15%, more preferred at least 20%, still more preferred at least 25%, still more preferred at least 30% of the amount of Factor V in the starting material. The eluted Factor V in preferred embodiments has a purity which is higher than 50%, preferably higher than 60%, more preferably higher than 70%, still more preferably higher than 80%, for instance about 90% or higher.

Thus, in certain exemplary embodiments, the invention provides a method for purifying Factor V from a biological fluid, the method comprising a step of bringing a biological fluid comprising Factor V into contact with an anion exchanger, washing the anion exchanger to remove contaminants, and eluting Factor V by treating the anion exchanger with a buffer containing between 0.35 M and 1 M NaCl (depending on the type of anion exchanger) to obtain a preparation further enriched in Factor V. The method further comprises a step of bringing said preparation comprising Factor V into contact with an immuno-affinity matrix containing anti-Factor V antibodies to bind Factor V to said matrix, washing the matrix to remove contaminants and eluting Factor V to obtain a preparation further enriched in Factor V. The purity of Factor V in said purified Factor V preparation is preferably at least 90%, more preferably at least 95%.

Alternatively, the anion exchange step of the invention could be followed by a dilution step performed prior to the innuno-affinity step, for instance to lower the salt concentration of the Factor V preparation in order to maximize Factor V binding to the immuno-affinity matrix.

According to the present invention, further purification steps may be performed after elution, following the immuno-affinity step. For example, the preparation can be, without limitations, further concentrated using conventional methods prior to buffer exchange to a final 50%-glycerol-based storage buffer or buffer exchange to a 5%-glycerol-based buffer and than freeze dried to obtain a final concentrated sample with a concentration of 50 % glycerol.

It will be clear that different embodiments of the invention can be combined, for instance a method is provided wherein the anion echange step is performed with a filter and wherein the following immuno-affinity step is performed with a column containing a matrix to which Epoxide functional groups are bound. Also, a method is provided wherein the anion exchange step is performed with a chromatographic monolith and wherein the following immuno-affinity step is performed with a filter.

The practice of this invention will employ, unless otherwise indicated, conventional techniques of immunology, molecular biology, microbiology, cell biology, recombinant DNA, and protein purification, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988; Bioprocess Technology vol 9. Separation Processes in Biotechnology (Marcel Dekker, Inc), Juan A Asenjo, ed, 1990.

The invention is further explained in the following examples. The examples do not limit the invention in any way. They merely serve to clarify the invention.

### Examples

### Example 1: Factor V purification based on research-grade protocol described by Bos et al. (comparative example)

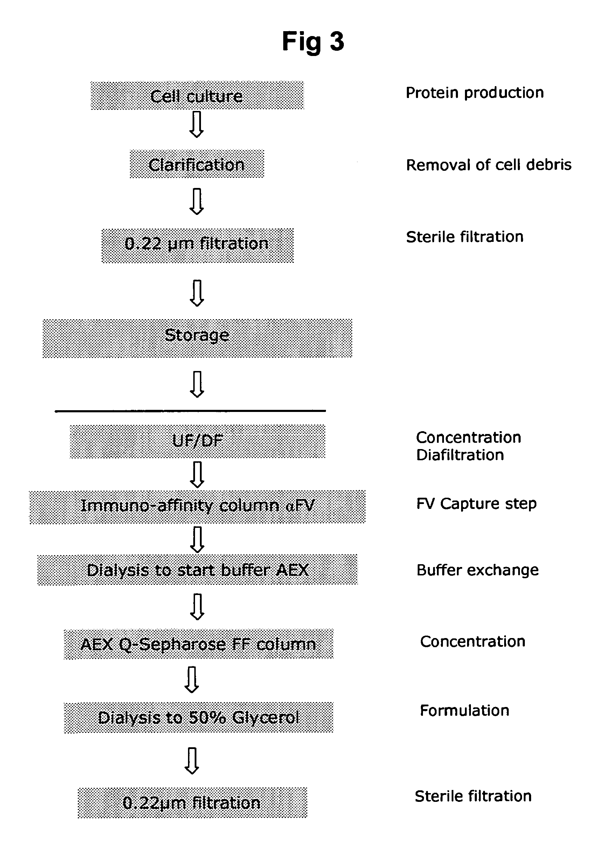

Wild type Factor V-L/C was recombinantly produced in adherently cultured PER.C6^{®}- FV-L/C wt cells, in roller bottles (production medium: DMEM + 2.5% Heat inactivated calf serum). The protocol described by Bos *et al* was implemented in a slightly adapted form. A process flow diagram is represented on FIG. 3. On day 1 an Ultrafiltration/ Diafiltration (UF/DF) step with a 100 kD 0.1 sq.m Pellicon-2 membrane from Millipore was implemented as alternative to the concentration step by Hemoflow F5 (Fresenius, Bad Homberg, Germany) described by Bos *et al.* During the UF/DF process, the harvest was concentrated 10 times over a 0.3 m² 100 KD Millipore Pellicon 2 membrane (Cat#:P2C100C01) and the concentrated material was dialysed against 3 volumes of buffer. On day 2, the immuno-affinity was performed using standard chromatography equipment (columns/ Fast Perfomance Liquid Chromatography) instead of using the αFV-NHS (N-hydroxysuccinimide) sepharose affinity resin from GE-Healthcare and was followed by dialysis to binding buffer (for anion exchange), which was performed overnight. On day 3, anion exchange chromatography was performed using Q-sepharose FF resin from GE Healthcare, again followed by dialysis performed overnight to the 50% glycerol-based storage buffer. On Day 4 the material was sterile filtered, aliquots were made and the material was stored at -20°C.

Factor V was obtained with this 4-days purification process. The presence of Factor V after purification was confirmed by SDS-page analysis (not shown). The overall recovery of the process was approximately 10-20% (based on ELISA).

This protocol, which is suitable for research batch productions, has two main drawbacks: the long process time and the presence of non-scalable process steps, such as dialysis. Moreover, it includes a harsh elution step of the immuno affinity column at pH, 10, which has a deleterious effect on product quality and may also reduce the life span of the anti-FV antibodies (bound to the column), leading to an overall increase of the cost of goods. Harsh elution steps are preferably avoided in large scale operations.

### Example 2: Factor V purification process according to the invention (Anion exchange followed by Immuno-affinity).

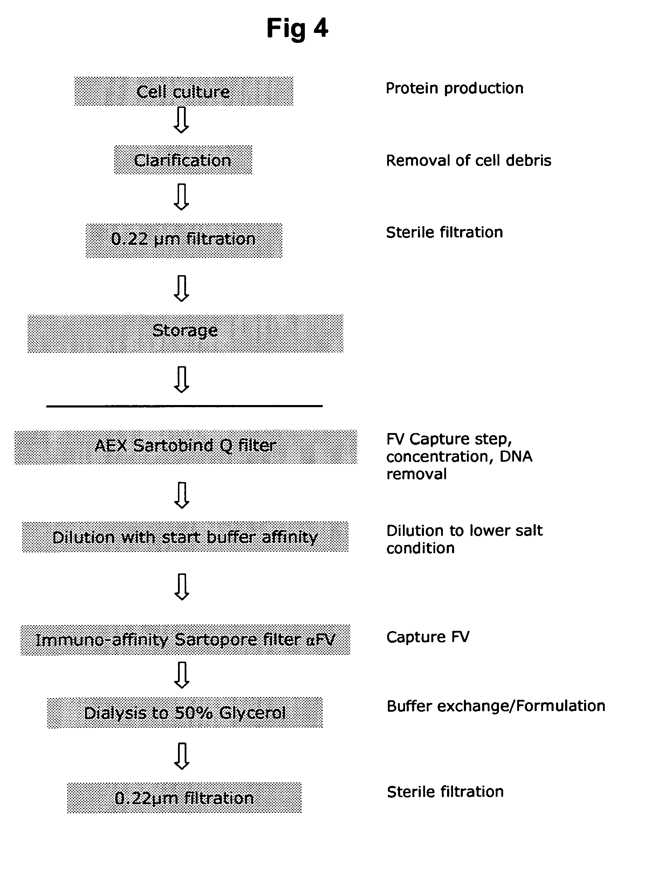

The anion exchange step and immuno affinity step were reversed as compared to the process described by Bos et al. Herewith, the intermediate dialysis (part of the UF/DF step) could be omitted. In addition, the resin containing columns (of the anion exchange step and the immuno affinity step) were replaced by filters, which allowed using higher flow rates, resulting in a reduced process time. A process flow diagram is represented on FIG. 4.

The adapted process started on day 1 with an anion exchange step using Sartobind Q filters (10K-15-25 Q filter with a bed volume of 280 ml from Sartorius) in binding mode. Elution of the Factor V was performed with 0.6 M NaCl buffer containing 10% glycerol, which gave the additional advantage of having DNA attached to the filter during elution and thus a cleaner Factor V preparartion for further purification. In order to remove DNA from the column, a buffer with increased ionic strength (higher NaCl concentration) was needed.

The eluate fraction of the anion exchanger was diluted 4 times with the start buffer of the Immuno-affinity step in order to reduce the salt concentration, which aids in maximal binding of Factor V to the immuno-affinity matrix. Subsequently, the process continued on day 1 or 2 with an immuno-affinity step using specific anti-FV antibody coupled to Sartobind Epoxy membranes (Sartobran 150 epoxy filter with a bed volume of 140 ml from Sartorius) followed by elution at pH10 with a buffer containing 50% ethylene glycol. Finally, a buffer exchange to the 50% glycerol-based storage buffer was performed.

Factor V was obtained with this 2-days purification process. The presence of Factor V after purification was confirmed by SDS-page analysis (not shown). The overall recovery of the process was approximately 10-20% (based on ELISA), which was similar to the process disclosed in Bos et al.

Surprisingly, reversing the order of the anion exchange step and the immuno affinity step resulted in similar process yields and a reduced process time (from 4 days to 2 days). An additional process improvement was the removal of host cell DNA (during the anion exchange step) prior to the immuno-affinity chromatography step, which led to less impurities/DNA loaded on the immuno-affinity filter. As a result, the filter is less likely to clog, and the number of cycles that the immuno-affinity membranes can be used for is increased, which in turn positively influences the cost of goods of the Factor V purification process.

### Example 3: Factor V purification process (use of chromatographic monoliths).

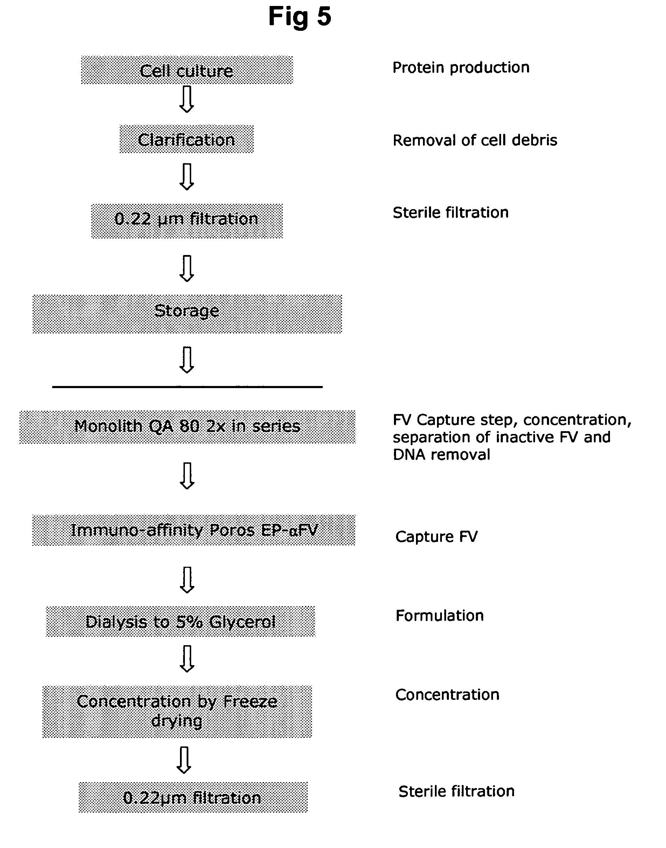

The purification protocol disclosed in example 2 was modified herein by using a chromatographic monolith instead of a filter during the anion exchange step and a column containing a cross-linked polystyrene-divinylbenzene matrix to which epoxide functional groups are bound (POROS® matrix) instead of a filter during the immuno affinity step. A process flow diagram is represented on FIG. 5.

The anion exchange step was performed using chromatographic monoliths containing quaternary amine groups (Monolith QA 80 ml from BIA separations with 2 columns mounted in series). The start buffer (binding buffer) consisted of 20 mM Tris.HCl, 200 mM NaCl, 1 mM CaCl₂ and 10 % Glycerol (pH 7.4). It was observed that when applying a two-step gradient, two eluate fractions were obtained. Surprisingly, the first eluate fraction, which was obtained with elution buffer 1 (360 mM NaCl) contained active Factor V. The second eluate fraction, which was obtained with elution buffer 2 (560 mM NaCl) contained inactive Factor V. Apparently, the high resolution of the monolith allowed for the exclusion of a non-active (possibly degraded or incompletely processed) fraction of Factor V. Herewith, a method was provided to obtain a Factor V preparation with an increased activity. DNA was also removed during this step.

The activity, which can be translated into the ability to induce clot formation was tested in a clot activity assay using FV-deficient human plasma. The eluate fractions were added to FV-deficient plasma (Dade Behring, Germany) employing normal human plasma as reference. Clotting was induced with Innovin^{®} (Dade Behring) or with Thromborel S (Dade Behring). Pooled plasma was again used as a standard. One unit of factor V activity was similar to the amount of FV in 1 mL of normal plasma (± 8 µg/mL).

The clot activity assay, which is based on the correlation between the Factor V concentration and the rate of clot formation allows for determining the Factor V concentration in the eluate fractions. The specific clot activity or RATIO (the ratio of active Factor V to the total Factor V fraction) was obtained by dividing the Factor V concentration (determined by the clot activity assay) with the total Factor V concentration (determined by ELISA). Tabel 1 shows the results of the clot activity assay for both the eluate fractions of Factor V obtained from the anion exchange step (using a monolith).

**Tabel 1. ELISA and clot activity assay**

| | ELISA (µg/ml) | CLOTTING ASSAY (µg/ml) | RATIO |
|---|---|---|---|
| Eluate fraction 1 | 57* | 41* | 0.7 |
| Eluate fraction 2 | 16* | < Detection level | NA |

| | | | |
|---|---|---|---|
| *Average ot three samples | | | |

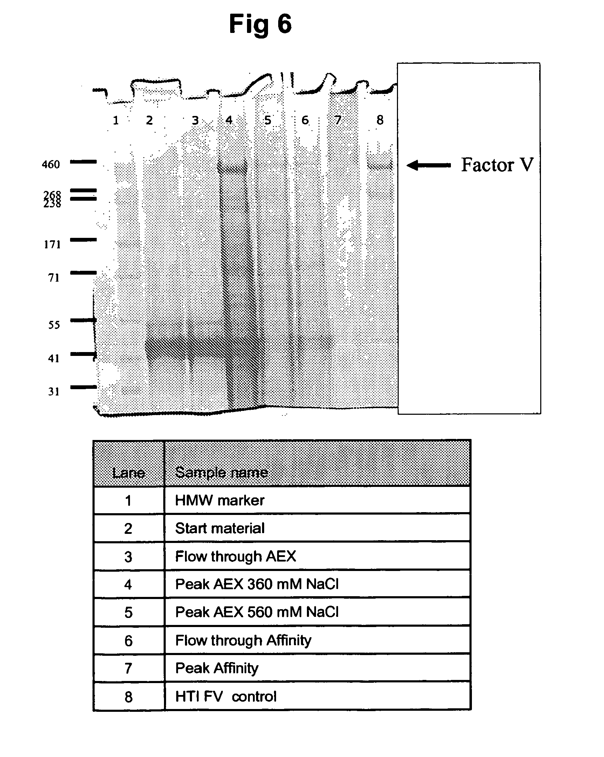

Eluate fraction 1 contained a 70% active Factor V preparation while Eluate fraction 2 had an undetectable activity. Factor V, which was clearly present in both eluate fractions (SDS-Page on FIG. 6) was surprisingly separated in an active and an inactive form with the use of a monolith.

An additional advantage of the monolith was the lower dead volume compared with the filter membranes resulting in a reduced dilution of the sample.

Importantly, the monolith can be run at equally high flow rates compared to the membranes. Due to the lower salt concentration of the first eluate fraction of the monolith, this sample does not have to be diluted before loading it onto the affinity column, thus reducing the loading time of the affinity column and keeping the sterility safe.

The immuno-affinity step was performed using an anti-FV antibody containing matrix based on 50 µm flowthrough particles made of a cross-linked polystyrene-divinylbenze to which epoxide functional surface groups are bound, from Applied biosystems (POROS® matrix).

Unexpectedly, the POROS® matrix has the advantage of less aspecifically binding impurities (compared to filter), resulting in a higher purity of the final preparation. Also, the dead volumes in the POROS® matrix are smaller then those in the filters and allow for higher peak concentrations. The specific binding and low dead volumes resulted in higher recovery yields. Moreover, the presence of rigid beads with big pore sizes in the POROS® matrix allowed for high flow capacity (similar to the flow in filters).

The Factor V was eluted from the column using high-salt treatment (buffer containing 2 M NaCl, 50 % Ethyleneglycol with a pH of 7.4), replacing the previously used pH10 elution buffer.

Optionally, if desired, the preparation can either be further concentrated using conventional methods prior to buffer exchange to the final 50%-glycerol-based storage buffer or buffer exchanged to a 5 %-glycerol-based buffer and than freeze dried to obtain a final concentrated sample with a concentration of 50 % glycerol.

The currently described protocol appeared to be very well suited for scale-up. Moreover, the current protocol is capable of delivering an active preparation of recombinant Factor V-L/C after two process steps with an over-all recovery of about 30% (based on ELISA), which is higher then the process disclosed in Bos et al.

### References

Bertina RM, Koeleman BP, Koster T, Rosendaal FR, Dirven RJ, de Ronde H, van der Velden PA, Reitsma PH. 1994 Nature 369 (6475): 14-5. Mutation in blood coagulation factor V associated with resistance to activated protein C.
Bos MH, Meijerman DW, van der Zwaan C, Mertens KJ. Does activated protein C-resistant factor V contribute to thrombin generation in hemophilic plasma? Thromb Haemost. 2005; 3:522-30.
Chan WP, Lee CK, Kwong YL, Lam CK, Liang R. A Novel Mutation of Arg306 of Factor V Gene in Hong Kong Chinese. Blood 1998;91:1135-39
M.E. Nesheim, K.H. Myrmel, L. Hibbard and K.G. Mann. Isolation and Characterization of single chain bovine factor V. JBC, Vol. 254, Issue 2, 508-517, Jan, 1979
van der Neut Kolfscholten M, Dirven RJ, Tans G, Rosing J, Vos HL, Bertina RM. The activated protein C (APC)-resistant phenotype of APC cleavage site mutant of recombinant factor V in a reconstituted plasma model. Blood Coagul Fibrinolysis 2002; 13:207-215
Svensson PJ, Dahlback B. Resistance to activated protein C as a basis for venous thrombosis. NEJM 1994;330:517-522
Williamson D, Brown K, Luddington R, Baglin C, Baglin T. 1998 Blood 91 (4): 1140-44. Factor V Cambridge: a new mutation (Arg306-->Thr) associated with resistance to activated protein C.

## Claims

1. A method for purifying Factor V from a biological fluid, said method comprising in the given order the steps of:
a) Binding factor V to an anion exchanger;
b) Washing the anion exchanger with a first solution to remove contaminants;
c) Eluting Factor V;
d) Specifically binding factor V to a matrix containing anti-Factor V antibodies;
e) Washing said matrix containing anti-Factor V antibodies with a second solution to remove contaminants; and
f) Eluting Factor V.

2. A method according to claim 1, wherein the anion exchanger in step a) is a filter.

3. A method according to claim 1, wherein the anion exchanger in step a) is a chromatographic monolith containing quaternary amine groups.

4. A method according to any one of claims 1-3, wherein the elution in step c) is performed by treating the anion exchanger with a buffer containing between 0.3 and 1 M NaCl buffer.

5. A method according to any one of claims 1-4, wherein the matrix of step d) is an immuno-affinity capture filter membrane.

6. A method according to any one of claims 1-4, wherein the matrix of step d) is a cross-linked polystyrene-divinylbenzene matrix to which Epoxide functional groups are bound.

7. A method according to any one of claims 1-6, wherein Factor V has been recombinantly expressed.

8. A method according to any one of claims 1-7, wherein Factor V is an APC-resistant Factor V mutant.

## Patentansprüche

1. Verfahren zur Aufreinigung von Faktor V aus einer biologischen Flüssigkeit, wobei das Verfahren die Schritte in der angegebenen Reihenfolge umfasst:
a) Binden von Faktor V an einen Anionenaustauscher;
b) Waschen des Anionenaustauschers mit einer ersten Lösung, um Kontaminanten zu entfernen;
c) Eluieren von Faktor V;
d) spezifisches Binden von Faktor V an eine Matrix, die Anti-Faktor-V-Antikörper enthält;
e) Waschen der Matrix, die Anti-Faktor-V-Antikörper enthält, mit einer zweiten Lösung, um Kontaminanten zu entfernen; und
f) Eluieren von Faktor V.

2. Verfahren nach Anspruch 1, wobei der Anionenaustauscher in Schritt a) ein Filter ist.

3. Verfahren nach Anspruch 1, wobei der Anionenaustauscher in Schritt a) ein chromatographischer Monolith ist, der quaternäre Amingruppen enthält.

4. Verfahren nach einem der Ansprüche 1 bis 3, wobei die Elution in Schritt c) durch Behandlung des Anionenaustauschers mit einem Puffer, der zwischen 0,3 und 1 M NaCl-Puffer enthält, durchgeführt wird.

5. Verfahren nach einem der Ansprüche 1 bis 4, wobei die Matrix in Schritt d) eine Immunoaffinitäts-Einfangfiltermembran ist.

6. Verfahren nach einem der Ansprüche 1 bis 4, wobei die Matrix in Schritt d) eine vernetzte Polystyrol-Divinylbenzol-Matrix ist, an die funktionelle Epoxidgruppen gebunden sind.

7. Verfahren nach einem der Ansprüche 1 bis 6, wobei Faktor V rekombinant exprimiert worden ist.

8. Verfahren nach einem der Ansprüche 1 bis 7, wobei Faktor V eine APCresistente Faktor-V-Mutante ist.

## Revendications

1. Procédé de purification du facteur V d'un liquide biologique, ledit procédé comprenant dans l'ordre donné les étapes de :
a) liaison du facteur V à un échangeur d'anions ;
b) lavage de l'échangeur d'anions avec une première solution pour retirer les substances contaminantes ;
c) élution du facteur V ;
d) liaison spécifiquement du facteur V à une matrice contenant des anticorps anti-facteur V ;
e) lavage de ladite matrice contenant des anticorps anti-facteur V avec une seconde solution pour retirer les substances contaminantes ; et
f) élution du facteur V.

2. Procédé selon la revendication 1, dans lequel l'échangeur d'anions dans l'étape a) est un filtre.

3. Procédé selon la revendication 1, dans lequel l'échangeur d'anions dans l'étape a) est un monolithe chromatographique contenant des groupes amine quaternaire.

4. Procédé selon l'une quelconque des revendications 1 à 3, dans lequel l'élution dans l'étape c) est effectuée en traitant l'échangeur d'anions avec une solution tampon contenant entre 0,3 et 1 M d'une solution tampon NaCl.

5. Procédé selon l'une quelconque des revendications 1 à 4, dans lequel la matrice de l'étape d) est une membrane filtre de capture par affinité immunologique.

6. Procédé selon l'une quelconque des revendications 1 à 4, dans lequel la matrice de l'étape d) est une matrice de polystyrène-divinylbenzène réticulée à laquelle des groupes fonctionnels époxyde sont liés.

7. Procédé selon l'une quelconque des revendications 1 à 6, dans lequel le facteur V a été exprimé de manière recombinante.

8. Procédé selon l'une quelconque des revendications 1 à 7, dans lequel le facteur V est un mutant du facteur V résistant à l'APC.
